(19) Europäisches Patentamt
European Patent Office
Office européen des brevets

(11) EP 1 586 639 A1

(12) **EUROPEAN PATENT APPLICATION**
published in accordance with Art. 158(3) EPC

(43) Date of publication:
**19.10.2005 Bulletin 2005/42**

(21) Application number: **02760765.4**

(22) Date of filing: **28.08.2002**

(51) Int Cl.[7]: **C12N 15/09**, C12Q 1/68, G01N 33/50

(86) International application number:
**PCT/JP2002/008670**

(87) International publication number:
**WO 2004/011643 (05.02.2004 Gazette 2004/06)**

(84) Designated Contracting States:
**DE ES FR GB GR IT SE**

(30) Priority: **26.07.2002 JP 2002218644**

(71) Applicant: **KABUSHIKI KAISHA TOSHIBA**
**Tokyo 105-8001 (JP)**

(72) Inventors:
- **TAKAHASHI, Masayoshi**
  **Yokohama-shi, Kanagawa 223-00 (JP)**
- **OKADA, Jun**
  **Yokohama-shi, Kanagawa 223-00 (JP)**
- **HASHIMOTO, Koji**
  **Atsugi-shi, Kanagawa 243-0813 (JP)**

(74) Representative: **Smyth, Gyles Darren**
**Marks & Clerk**
**90 Long Acre**
**London WC2E 9RA (GB)**

(54) **NUCLEIC ACID PROBE IMMOBILIZATION SUPPORT AND METHOD OF DETECTING TARGET NUCLEIC ACID USING THE SAME**

(57) The invention provides a nucleic acid probe-immobilized substrate comprising a substrate and a nucleic acid probe containing a nucleotide sequence complementary to a target sequence and immobilized via a spacer onto the substrate, wherein upon hybridization, with the nucleic acid probe, of a target nucleic acid partially containing the target sequence, the spacer satisfies the relationship:

$$X \geqq Y$$

wherein X is the length of the spacer and Y is the length of the target nucleic acid ranging from the end of the hybridized site at the side of the substrate to the end of the target nucleic acid at the side of the substrate.

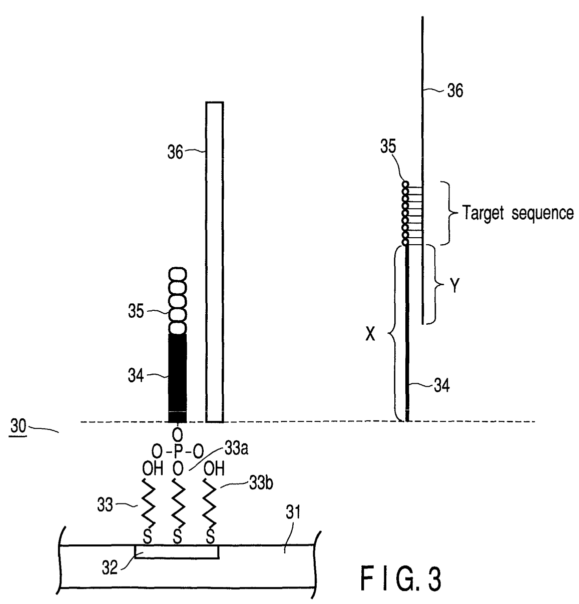


F I G. 3

EP 1 586 639 A1

## Description

Technical Field

The present invention relates to a nucleic acid probe-immobilized substrate for detecting the presence of target nucleic acid and a method of detecting nucleic acid by using the same.

Background Art

As genetic engineering has developed in recent years, genetic diagnosis and prevention of diseases has been made feasible in the field of medical treatment. This is called genetic diagnosis. For example, a certain disease can be diagnosed or predicted before the onset of the disease or at a very early stage by detecting a defect or change in a human gene causing the disease. As studies of the relationship of genotypes to diseases together with the decoding of the human genome advance, treatment adapted to the genotype of each individual (tailor-made medical treatment) is being realized. Accordingly, it is very important to facilitate detection of a gene or determination of a genotype.

A device generally called a DNA chip or a DNA microarray (referred to collectively as a DNA chip) is attracting attention in such genetic analysis. The DNA chip is a device comprising a large number of nucleic acid probes consisting of many kinds of nucleotide sequence immobilized on a substrate. By using the DNA chip, many kinds of target nucleic acid can be detected in a single test. While having the advantage described above, the DNA chip shows varying efficiency of hybridization with the different target nucleic acids present in a sample, and in some cases the efficiency of hybridization may be very low.

Disclosure of Invention

In light of the circumstances described above, an object of the present invention is to provide a probe-immobilized substrate capable of effecting highly efficient hybridization.

This object can be achieved by the following aspects of the invention:

(1) a nucleic acid probe-immobilized substrate comprising a substrate and a nucleic acid probe containing a nucleotide sequence complementary to a target sequence and immobilized via a spacer onto the substrate, wherein upon hybridization, with the nucleic acid probe, of a target nucleic acid partially containing the target sequence, the spacer satisfies the relationship:

$$X \geqq Y$$

wherein X is the length of the spacer and Y is the length of the target nucleic acid ranging from the end of the hybridized site at the side of the substrate to the end of the target nucleic acid at the side of the substrate;

(2) a method of detecting the presence of a target nucleic acid by use of the nucleic acid probe-immobilized substrate described in item (1) above, the method comprising:

a step of amplifying a nucleic acid sample with primers selected so as to satisfy the relationship $X \geqq Y$;
a step of allowing the amplification product obtained by the amplification to react, under conditions achieving suitable hybridization, with a nucleic acid probe immobilized on the nucleic acid probe-immobilized substrate described in item (1) above; and
a step of detecting the hybridization occurring in the above reaction, thereby determining that the target nucleic acid is present in the nucleic acid sample; and

(3) a method of detecting a target nucleic acid containing a target sequence by use of a nucleic acid probe-immobilized substrate comprising a substrate and a nucleic acid probe containing a sequence complementary to the target sequence and immobilized onto the substrate, the method comprising:

(a) preparing primers for amplifying the target nucleic acid such that, upon hybridization of the target sequence in the target nucleic acid with the nucleic acid probe, the end of the target nucleic acid is located within 40 bases from the end of the target sequence site at the side of the substrate,
(b) amplifying a nucleic acid sample with the primers prepared in the step (a) above;
(c) allowing the amplification product obtained in the step (b) above to be single-stranded;
(d) allowing the single strand obtained in the step (c) above to react with the nucleic acid probe; and

(e) detecting the hybridization occurring in the step (d) above, thereby detecting the presence of the target nucleic acid in the nucleic acid sample.

Brief Description of Drawings

FIG. 1A is a plan view showing one example of a nucleic acid probe-immobilized substrate of the invention, and FIG. 1B is a sectional view of FIG. 1A taken along the line B-B.
FIG. 2 is a drawing showing one example of the nucleic acid probe-immobilized substrate of the invention.
FIG. 3 is a drawing showing a state of the nucleic acid probe bound to target nucleic acid.
FIG. 4 is a drawing schematically illustrating the nucleic acid probes and target nucleic acids used in the Example.
FIG. 5 is a graph showing the relationship between X and Y.
FIG. 6A is a drawing showing a state of binding of nucleic acid probes containing various spacers to a target nucleic acid used in the Example, and FIG. 6B is a graph showing the results obtained in a test carried out in the Example.
FIG. 7A is a drawing showing a state of binding of nucleic acid probes containing various spacers to a target nucleic acid used in the Example, and FIG. 7B is a graph showing the results obtained in a test carried out in the Example.
FIG. 8A is a drawing showing a state of binding of nucleic acid probes containing various spacers to a target nucleic acid used in the Example, and FIG. 8B is a graph showing the results obtained in a test carried out in the Example.
FIG. 9 is a drawing showing the relationship between the amplification fragment and nucleic acid probe used in the Example.
FIG. 10 is a graph showing the results obtained in a test carried out in the Example.
FIG. 11 is a drawing showing the relationship between the amplification fragment and nucleic acid probe used in the Example.
FIG. 12 is a graph showing the results obtained in a test carried out in the Example.
FIG. 13 is a graph showing the results obtained in a test carried out in the Example.

Best Mode for Carrying Out of the Invention

1. Summary of Invention

This invention relates to a nucleic acid probe-immobilized substrate consisting essentially of a substrate and a nucleic acid probe immobilized via a spacer onto the substrate. This invention is based on the inventors' finding that more efficient hybridization can be achieved by specifying the length of the spacer.

That is, the nucleic acid probe-immobilized substrate according to an embodiment of the invention is constituted such that upon hybridization, with the nucleic acid probe, of a target nucleic acid partially containing a target sequence, the nucleic acid probe will have been immobilized on the substrate via a spacer satisfying the relationship $X \geqq Y$ wherein X is the length of the spacer and Y is the length of the target nucleic acid ranging from the end of the hybridized site at the side of the substrate to the end of the target nucleic acid at the side of the substrate.

The principle of measurement by the nucleic acid probe-immobilized substrate according to an aspect of this invention is as follows. The nucleic acid probe is designed to have a sequence complementary to a target sequence. When a nucleic acid sample contains the target sequence, there occurs hybridization on the nucleic acid probe-immobilized substrate. It follows that, fundamentally, the device of the invention detects the presence of a double-stranded nucleic acid formed as a result of the hybridization or the presence of nucleic acid hybridized with the nucleic acid probe, thereby enabling detection of the presence of the target sequence in the nucleic acid sample. As used herein, the phrase "detection of hybridization" refers collectively to detection of formed double-stranded nucleic acid, detection of a signal after hybridization, the signal being derived from a label used in labeling a nucleic acid sample, and detection, by another means known per se, of the presence of a double-stranded nucleic acid formed by reaction or the occurrence of hybridization.

Such detection of hybridization can be carried out, for example, by electrochemical detection or fluorescence detection described later.

In the case of electrochemical detection, the nucleic acid probe-immobilized substrate can be obtained fundamentally by immobilizing a desired nucleic acid probe via a spacer onto an electrode arranged on a substrate so as to enable detection of an electrochemical signal.

In the case of detection by means of using a label such as a fluorescent substance, the nucleic acid probe-immobilized substrate can be obtained fundamentally by immobilizing a desired nucleic acid probe via a spacer onto a substrate.

2. Description of Terms

As used herein, the term "nucleic acid" refers collectively to nucleic acids and nucleic acid analogues such as ribonucleic acid (that is, RNA), deoxyribonucleic acid (that is, DNA), peptide nucleic acid (that is, PNA), methylphosphonate nucleic acid, S-oligo, cDNA, cRNA, oligonucleotide and polynucleotide. The nucleic acid may be a naturally occurring or artificially synthesized nucleic acid.

As used herein, the "nucleic acid probe" is a nucleic acid containing a nucleotide sequence complementary to a target sequence, and refers to a nucleic acid fragment to be immobilized on a substrate. The nucleic acid probe has a sequence complementary to the intended target sequence, through which the probe can hybridize with the target sequence under suitable conditions.

As used herein, the term "target sequence" refers to a nucleotide sequence whose presence is to be detected or a sequence to be captured by the nucleotide sequence of the nucleic acid probe. The nucleic acid containing the target sequence is called target nucleic acid.

As used herein, the term "complementary" refers to being complementary in the range of 50% to 100%, preferably 100%.

As used herein, the term "spacer" refers to a linear substance having a certain length arranged between the nucleic acid probe and the substrate. When the substance constituting the spacer is nucleic acid, a portion complementary to or hybridized with the target sequence is classified as the probe and the other portion as the spacer.

As used herein, the term "length of the spacer" refers to the length of the linear molecule arranged between the nucleic acid probe and the substrate. When a blocking agent shown in FIG. 3 is used on the substrate, the "length of the spacer" is the length of the spacer minus the length of the blocking agent.

3. Mode of the Invention

First, the fundamental constitution of this invention is described below.

(1) First embodiment

A first embodiment of the invention is described by reference to FIG. 1. In the first embodiment of the invention, the nucleic acid probe-immobilized substrate 1 comprises a nucleic acid probe 5 immobilized via a spacer 4 on an electrode 3 arranged on a substrate 2 (FIGS. 1A and 1B). The electrode 3 is connected to a pad 6 for retrieving electrical information. In FIG. 1B, the spacer 4 is expressed in a thick line, and the nucleic acid probe 5 is expressed in a chain-like line, for convenience' sake.

The nucleic acid probe-immobilized substrate 1 can be produced by arranging an electrode on a silicon substrate by means known per se and then immobilizing a nucleic acid probe via a spacer on the surface of the electrode.

The number of electrodes in this embodiment was 6, but the number of electrodes arranged on one substrate is not limited thereto. Further, the pattern of arrangement of electrodes is not limited to that of FIG. 1A, and the design can be suitably modified as necessary by those skilled in the art. A reference electrode and a counter electrode may be provided if necessary. Such a nucleic acid probe-immobilized substrate also falls under the scope of this invention.

(2) Second embodiment

A second embodiment of the invention is illustrated in FIG. 2. In the second embodiment of the invention, a nucleic acid probe-immobilized substrate 11 comprises a nucleic acid probe 14 immobilized via a spacer 13 on a substrate 12 (FIG. 2). In FIG. 2, the spacer 13 is expressed in a thick line and the nucleic acid probe 14 is expressed in a chain-like line, for convenience' sake.

The nucleic acid probe-immobilized substrate 11 can be produced for example by arranging a nucleic acid probe via a spacer on a silicon substrate by means known per se.

In this embodiment, the number of electrodes to be arranged on one substrate is not limited thereto and may be changed if desired, or nucleic acid probes having plural kinds of nucleotide sequences may be arranged on one substrate. The solid-phase pattern of plural nucleic acids and/or plural kinds of nucleic acid on a substrate can be suitably modified as necessary by those skilled in the art. Such a nucleic acid probe-immobilized substrate also falls under the scope of this invention.

4. Constitution

The embodiment of the invention with the fundamental constitution described above is characterized in that the nucleic acid probe is immobilized via a spacer. The spacer used in this invention is specifically a spacer wherein upon hybridization, with the nucleic acid probe, of a target nucleic acid partially containing the target sequence, the spacer satisfies the relationship X ≧ Y wherein X is the length of the spacer and Y is the length of the target nucleic acid ranging from the end of the hybridized site at the side of the substrate to the end of the target nucleic acid at the side of the substrate.

FIG. 3 shows one example of a state of the nucleic acid probe bound to a target nucleic acid. A nucleic acid probe-immobilized substrate 30 and a general target nucleic acid are illustrated on the left in FIG. 3. A surface of an electrode 32 arranged on a substrate 31 is treated with a linker agent 33a and a blocking agent 33b. A nucleic acid probe 35 is immobilized via a spacer 34 on the electrode 32. A target nucleic acid 36 partially containing a target sequence complementary to the sequence of the nucleic acid probe 35 is also shown.

Even if a nucleic acid sample obtained from a target, such as an individual, tissues and cells or a sample obtained therefrom after desired treatment, is a target nucleic acid containing a target sequence to be detected, it is considered that the position of the target sequence in the target nucleic acid is varied. The left nucleic acid sample 36 in FIG. 3 shows an average example of such various target nucleic acids.

The state of the thus immobilized nucleic acid probe 35 hybridized with the target nucleic acid 36 is shown on the right in FIG. 3 to compare the upper portion over a base line 37. As is evident from the drawing, "X" is the length of the spacer 34, and "Y" is the length of the target nucleic acid ranging from the end of the hybridized site at the side of the substrate to the end of the target nucleic acid at the side of the substrate upon hybridization of the target nucleic acid 36 via the target sequence with the nucleic acid probe 35.

When X < Y, the efficiency of hybridization between the nucleic acid probe 35 having a sequence complementary to the target sequence and the target nucleic acid 36 is low. That is, in consideration of the length of the target nucleic acid and the position of the target sequence in the target nucleic acid, the nucleic acid probe immobilized is too close to the surface of the substrate in this case. Accordingly, the nucleic acid probes can cause steric hindrance, or the solid-phase substrate can cause steric hindrance. As a result, the efficiency of hybridization of the nucleic acid probe with the target nucleic acid cannot be high.

On the other hand, when X ≧ Y, the efficiency of hybridization between the nucleic acid probe 35 having a sequence complementary to the target sequence and the target nucleic acid 36 is high. As is evident from the right drawing in FIG. 3, when the target probe is hybridized with the target sequence in the case of X ≧ Y, an excess, if any, of the target nucleic acid 36 extending from the target sequence toward the substrate 31 is short, or such excess is not present. In consideration of the length of the target nucleic acid and the position of the target sequence, the spacer 34 is considered to be sufficiently long, and thus the nucleic acid probe can move freely in a broad range in a reaction solvent (that is, the degree of freedom is high) to increase the probability at which the nucleic acid probe encounters the target sequence during the hybridization reaction.

The relationship between X and Y is shown in FIG. 5. In the graph in FIG. 5, the length X is shown on the abscissa, while the length Y is shown on the ordinate. The central straight line is a graph of Y = X. According to the embodiment of the invention, the relationship between Y and X is preferably X ≧ Y. In FIG. 5, region A is a region satisfying X ≧ Y in order to reduce the steric hindrance between the target nucleic acid and the solid phase. Region B is a region contained in region A and satisfying X-50 Å ≧ Y in order to further improve the degree of freedom of the nucleic acid probe. Region C is a region contained in region B and desired in consideration of the cost and yield in synthesis of the nucleic acid probe. Region D is a region preferably avoided in consideration of necessity for at least a certain length of X to secure the degree of freedom even if Y is shorter than several tens of Å. Further, region E is a region not influencing the efficiency of hybridization regardless of the presence, absence or length of the spacer because Y is 0 or very short.

When detection is actually conducted, regions A, B, C and D can be often used, but regions C and D are more preferable regions in the embodiments of the invention.

According to the principles of this invention, the limit of the length X may be 20000 Å or less, or 10000 Å or less. From the viewpoint of synthesis of the nucleic acid probe, the upper limit of the length X may be 2000 Å (which in terms of nucleic acid, corresponds to about 400 bases), preferably 1000 Å, and more preferably 500 Å. This is because when the length X is determined to be long, there is the possibility that the yield and purity in synthesis of the nucleic acid probe may be lowered.

To satisfy the conditions in accordance with the embodiments of the invention described above, it is also possible to devise selection of the target sequence or selection of the sequences of primers used in amplification of the target nucleic acid containing the target sequence from a sample. Higher efficiency of hybridization can be achieved not only by regulating the length of the spacer but also by such regulation.

The material which can be used as the spacer may be organic linear molecules, for example nucleic acid, alkane, polyethylene glycol, polypeptide, etc.

For example, when the spacer is a nucleic acid spacer consisting of nucleic acid, the nucleotide sequence thereof is preferably a sequence which does not bind to the target nucleic acid or nucleic acid contained in the sample. Further, when occurring hybridization is detected by electrochemical detection described later, the sequence of the spacer is determined preferably by considering the tendency of binding to the nucleic acid bases of a double strand-recognizing body used. For example, Hoechst 33258 hardly binds to cytosine or guanine, but easily binds to thymine or adenine. On one hand, a sequence of contiguous guanine residues is hardly synthesized. Accordingly, a nucleotide sequence containing only cytosine residues or many cytosine residues is more preferable, a nucleotide sequence consisting of thymine residues or containing many thymine residues is preferable, and a nucleotide sequence containing guanine or adenine residues only or a large number of these residues is not preferable.

By arrangement of such spacer, efficient hybridization between the nucleic acid probe and the target nucleic acid can be achieved.

The nucleic acid probe used in this invention may have a length used generally for a probe. For example, the length of the nucleic acid probe may be about 3-base length to about 1000-base length, preferably about 10- to about 200-base length.

The substrate which can be used in this invention may be any substrate on which the nucleic acid probe to be hybridized with the target sequence can be immobilized. Such substrate may be for example a nonporous, rigid or semi-rigid material, and may be in a plate having wells, grooves or a flat surface or in a three-dimensional spherical or cubic form. The substrate includes, but is not limited to, silica-containing substrates such as silicon and glass and substrates produced from plastics and polymers such as polyacrylamide, polystyrene and polycarbonate. However, an electrode itself described later may be used as the substrate in place of the above-described substrates.

In the case of the nucleic acid probe-immobilized substrate to be subjected to fluorescence detection, the nucleic acid probe may be immobilized via a space on any of the substrates described above. In the case of the nucleic acid probe-immobilized substrate to be subjected to electrochemical detection, an electrode is arranged on any of the substrates described above so as to enable electrochemical detection, and the nucleic acid probe may be immobilized on the electrode.

The electrode which can be used in this invention is not particularly limited, and examples thereof include carbon electrodes such as graphite, grassy carbon, pyrolytic graphite, carbon paste and carbon fiber, a noble metal electrode such as platinum, platinum black, gold, palladium and rhodium, an oxide electrode such as titanium oxide, tin oxide, manganese oxide and lead oxide, and a semiconductor electrode such as Si, Ge, ZnO, CdS, $TiO_2$ and GaAs, titanium, etc. These electrodes may be coated with an electrically conductive polymer or a monomolecular film, and if necessary treated with another surface treating agent.

Immobilization of the nucleic acid via the spacer may be carried out by means known per se. For example, the spacer is immobilized on the electrode, and thereafter, the nucleic acid probe may be immobilized on the spacer. Alternatively, the spacer may be previously bound to the nucleic acid probe, and the nucleic acid probe may be immobilized via the spacer on the electrode. Alternatively, the spacer and the nucleic acid probe may be synthesized on the electrode by means known per se. Further, immobilization of the nucleic acid probe via the spacer may be carried out by directly immobilizing the spacer via covalent bonding, ionic bonding or physical adsorption onto the treated or untreated substrate or the surface of the electrode. Alternatively, a linker agent facilitating immobilization of the nucleic acid probe via the spacer may be used, and such a linker agent may be used to immobilize the nucleic acid probe via the spacer on the substrate or the electrode. Further, a blocking agent for preventing nonspecific binding of the nucleic acid probe to the electrode, together with the linker agent, may be used to treat the electrode. The linker agent and blocking agent used may be materials for advantageously carrying out electrochemical detection.

Further, the nucleic acid probes having different nucleotide sequences may be immobilized via spacers on different electrodes, or a mixture of plural kinds of nucleic acid probe having different nucleotide sequences may be immobilized via spacers onto one electrode.

5. Detection

The nucleic acid probe-immobilized substrate according to this invention can utilize an electrochemical method and a fluorescence detection method as a means for detecting the presence of a double strand occurring as a result of a hybridization reaction between the nucleic acid probe immobilized on the substrate and the target nucleic acid.

(1) Electrochemical detection

Electrochemical detection of the double-stranded nucleic acid may be carried out, for example, by using a double strand-recognizing substance known per se.

For example, the double strand-recognizing substance includes, but is not limited to, bisintercalators, trisintercalators and polyintercalators such as Hoechst 33258, acridine orange, quinacrine, daunomycin, metallointercalator and bisacridine. Further, these intercalators may be modified with an electrochemically active metal complex such as ferrocene and biologen. Any other known double strand-recognizing substances can also be preferably used in this invention.

The nucleic acid probe-immobilized substrate according to this invention comprises a nucleic acid probe immobilized via a spacer on an electrode. For detection of double-stranded nucleic acid by the electrode, a counter electrode and a reference electrode may further be used in the same manner as in other general methods of electrochemical detection. When a reference electrode is arranged, a general reference electrode such as a silver/silver chloride electrode and a mercury/mercury chloride electrode may be used.

For example, the following test may be carried out to determine whether or not the target nucleic acid is contained in the nucleic acid sample. For example, a nucleic acid component is extracted as the nucleic acid sample from a sample collected from a subject such as animal individuals including humans, tissues and cells. The resultant nucleic acid sample is subjected if necessary to treatment such as reverse transcription, elongation, amplification and/or enzyme treatment. The pre-treated nucleic acid sample is brought into contact with the nucleic acid probe immobilized on the nucleic acid probe-immobilized substrate and allowed to react under conditions where suitable hybridization can occur. Such suitable conditions can be suitably selected by those skilled in the art, depending on various conditions such as the types of base contained in the target sequence, the type of spacer and nucleic acid probe to be arranged on the nucleic acid probe-immobilized substrate and the type of nucleic acid sample, as well as the states thereof. The conditions for the reaction include, but are not limited to, the following conditions.

That is, the hybridization reaction is carried out in a buffer solution with an ionic strength in the range of 0.01 to 5 and in the range of pH 5 to 10. A hybridization promoter such as dextran sulfate or salmon sperm DNA, bovine thymus DNA, EDTA and a surfactant may be added to this solution. The nucleic acid component obtained above is added thereto and thermally denatured at 90°C or more. Addition of the thermally denatured nucleic acid sample to the nucleic acid probe-immobilized substrate may be carried out just after denaturation or after quenching to 0°C. Alternatively, the hybridization reaction may be carried out by dropping the solution onto the substrate.

During the reaction, the reaction rate may be increased by a procedure such as stirring or shaking. The reaction may be carried out at a temperature in the range of 10°C to 90°C for 1 minute to overnight. After the hybridization reaction, the electrode is washed. For example, a buffer solution with an ionic strength in the range of 0.01 to 5 and in the range of pH 5 to 10 may be used in washing. When the target nucleic acid containing the target sequence is present in the nucleic acid sample, the target nucleic acid is hybridized with the nucleic acid probe, to generate a double-stranded nucleic acid.

Subsequently, the double-stranded nucleic acid thus generated is detected by electrochemical means in the following procedure. Generally, the substrate after the hybridization reaction is washed, and a double strand-recognizing body is allowed to act on the double-stranded moiety formed on the surface of the electrode, and a signal generated therefrom is electrochemically measured.

The concentration of the double strand-recognizing body, though being varied depending on its type, is used generally in the range of 1 ng/mL to 1 mg/mL. For this reaction, a buffer solution with an ionic strength in the range of 0.01 to 5 and in the range of pH 5 to 10 may be used.

For example, electrochemical measurement may be carried out using a reaction current derived from the double strand-recognizing body upon application of a potential higher than a potential for the electrochemical reaction of the double chain-recognizing body. In this case, the potential may be swept at a constant rate or applied by pulsation, or a constant potential may be applied. For measurement, the electricity and voltage may be regulated by using a device such as potentiostat, a digital multimeter and a function generator. For example, the concentration of the target nucleic acid may be calculated on the basis of the determined electricity by using a calibration curve.

Further, electrochemical detection means known per se, for example means disclosed in the papers of Hashimoto et al. 1994 and Wang et al. 1998 can also be preferably used in the method of this invention. In these papers, Hashimoto et al. reported detection of a sequence-specific gene by using a gold electrode modified with a DNA probe and an electrochemically active coloring matter. The anodic electricity derived from the coloring matter is correlated with the concentration of target DNA. Further, Wang et al. reported indicator-free electrochemical DNA hybridization. The constitution of this biosensor includes immobilization of an inosine-substituted probe (not containing guanine) on a carbon paste electrode and chromopotentiometric detection of formation of a double-stranded chain by the presence of an oxidation peak of guanine in the label. The detection means described in these papers may be used preferably in this invention.

(2) Fluorescence detection method

In the method of using a fluorescence label, a nucleic acid sample can be labeled with a fluorescent coloring matter such as FITC, Cy3, Cy5 or rhodamine, an enzyme such as biotin, hapten, oxidase or phosphatase, or an electrochemically active substance such as ferrocene or quinone. Alternatively, detection is carried out with a second probe labeled with the above-described substance. A plurality of labels can also be used simultaneously.

In some embodiments, the reaction of a nucleic acid component extracted from a sample with the probe immobilized on the probe-immobilized chip is carried out, for example, in the following manner. Namely, the hybridization reaction is carried out in a buffer solution with an ionic strength in the range of 0.01 to 5 and in the range of pH 5 to 10. A hybridization promoter such as dextran sulfate or salmon sperm DNA, bovine thymus DNA, EDTA and surfactant may be added to this solution. An extracted nucleic acid component is added thereto and thermally denatured at 90°C or more. Addition of the thermally denatured nucleic acid sample to the nucleic acid probe-immobilized chip may be carried out just after denaturation or after quenching to 0°C. Alternatively, the hybridization reaction may be carried out by dropping the solution onto the substrate. During the reaction, the reaction rate may be increased by a procedure such as stirring or shaking. The reaction may be carried out at a temperature in the range of 10°C to 90°C for 1 minute to overnight. After the hybridization reaction, the substrate is washed. For example, a buffer solution with an ionic strength in the range of 0.01 to 5 and in the range of pH 5 to 10 may be used in washing.

In the case of fluorescence detection, detection of the hybridization reaction is carried out by detecting a labeled nucleotide sequence in a sample or a label in a secondary probe by means of a suitable detector adapted to the type of the label. When the label is a fluorescence material, the label may be detected, for example, by a fluorescence detector.

Further, the method of detecting the presence of any target nucleic acids or any target sequences by using the nucleic acid probe of the invention described above falls under the scope of this invention.

In particular, primers for achieving the above-described relationship between X and Y are used to amplify a nucleic acid sample, the resultant amplification product is reacted with the nucleic acid probe immobilized on the nucleic acid probe-immobilized substrate in accordance with the embodiments of this invention, and the occurring hybridization is detected, whereby the presence of the target nucleic acid can be detected and higher efficiency of hybridization can be achieved. This invention also encompasses such a method. Further, the amplification which can be used in such a method includes, for example, amplification such as polymerase chain reaction (generally called PCR and referred to hereinafter as PCR), reverse transcription PCR such as reverse transcription amplification using a reverse transcriptase, and other amplification known per se.

The amplification which can be used according to the embodiments of the invention includes, for example, nucleic acid strand amplification (NASBA), transcription mediated amplification (TMA), ligase chain reaction (LCR), strand displacement amplification (SDA), isothermal and chimeric primer-initiated amplification of nucleic acids (ICANN), rolling circle amplification (RCA), etc.

The method of analyzing nucleic acid according to the embodiments of this invention can be utilized in analysis of nucleic acid contained in a sample, for example, detection and quantification of the presence of a target sequence, expression analysis such as expression and disappearance of gene expression, analysis of polymorphism such as single nucleotide polymorphism (SNP) and microsatellite sequences in genome, diagnosis of diseases and prediction of the risk factor of onset by analysis of disease-related genes, detection of the presence of infections, analysis of virus type, or in order to carry out a toxic test. Accordingly, the method of the invention can be utilized for various clinical purposes such as clinical diagnosis and prediction of onset. Further, the method of the invention can be utilized widely in various fundamental studies or applied studies in examination of foods, quarantine inspection, examination of pharmaceutical preparations, legal medicine, farming, stockbreeding, fishery and forestry. Example

Hereinafter, the method of detecting nucleic acid according to this invention is described by reference to the Example.

In this example, the relationship between the length (X) of the spacer in the nucleic acid probe and the length (Y) of the target nucleic acid ranging from the nucleic acid probe-bound site to the end of the target nucleic acid at the side of the substrate, and the relationship with the efficiency of hybridization, were examined.

(1) Relationship between the nucleic acid probe and the target nucleic acid

The relationship between the nucleic acid probes and the target nucleic acids used in this example is shown in FIG. 4. The specific sequences of the nucleic acid probes and the target nucleic acids will be described later, and first, an approximate constitution thereof and correlation are described.

FIG. 4 shows nucleic acid probes C-0, C-10, C-20 and C-30 and target nucleic acids 70-0, 70-20 and 70-40 whose target sequences and its complementary sequences, each consisting of 20 bases, are arranged side by side.

Four kinds of nucleic acid probe were used. The sequence complementary to the target sequence in the nucleic acid probe is a 20-base sequence which corresponds to the portion with slants in FIG. 4.

The nucleic acid probe C-0 does not have a spacer at the 5-terminus thereof.

The nucleic acid probe C-10 has spacer X1 consisting of 10 cytosine bases added to the 5'-terminus thereof.

The nucleic acid probe C-20 has spacer X2 consisting of 20 cytosine bases added to the 5' terminus thereof.

The nucleic acid probe C-30 has spacer X3 consisting of 30 cytosine bases added to the 5' terminus thereof. These 4 nucleic acid probes are identical with one another except for the spacer. Further, any of these nucleic acid probes are immobilized via the 5'-terminus on a substrate.

Three kinds of target nucleic acid were used. Target sequences in these 3 target nucleic acids have the same length, i.e. 20 bases. In FIG. 4, the target sequence corresponds to the netted portion. The target sequences contained in the 3 target sequences have the same nucleotide sequence.

The target nucleic acid 70-0 is a nucleic acid having a full length of 70 bases with the target sequence of 20 bases present at the 3'-terminus thereof.

The target nucleic acid 70-20 is a nucleic acid having a full length of 70 bases. 30 bases are present at the 5'-side of the target sequence, while sequence Y1 of 20 bases is present at the 3'-side of the target sequence.

The target nucleic acid 70-40 is a nucleic acid having a full length of 70 bases. 10 bases are present at the 5'-side of the target sequence, while sequence Y2 of 40 bases is present at the 3'-side of the target sequence.

(2) Nucleic acid probes

The sequence which in the nucleic acid probe, is complementary to the target sequence is a 20-base sequence. The nucleic acid probes C-0, C-10, C-20 and C-30 are those probes having 0, 10, 20 and 30 C (cytosine) bases added as a spacer to the 5'-terminus of the 20-base nucleic acid probe sequence. Their sequences are as follows.

C-0: 5′-SH-TGGACGAAGACTGACGCTC-3′ (SEQ ID NO:1)

C-10: 5′-SH-($C_{10}$)TGGACGAAGACTGACGCTC-3′ (SEQ ID NO:2)

C-20: 5′-SH-($C_{20}$)TGGACGAAGACTGACGCTC-3′ (SEQ ID NO:3)

C-30: 5′-SH-($C_{30}$)TGGACGAAGACTGACGCTC-3′ (SEQ ID NO:4)

Each of the 4 probes, i.e. C-0, C-10, C-20 and C-30, has been modified with a thiol group at the 5'-terminus thereof. Further, the X moieties in the nucleic acid probes C-0, C-10, C-20 and C-30 are 0-, 10-, 20- and 30-base sequences respectively.

(3) Target nucleic acids

As a model of the target nucleic acid, a 70-base oligonucleotide containing a sequence complementary to the above 20-base sequence was separately prepared. As that moiety of the target nucleic acid which ranges from the terminus of the probe-binding site to the 3'-terminus thereof, 3 sequences of 0, 20 and 40 bases in length were used. The respective sequences are as follows.

Target nucleic acid 70-0 (SEQ ID NO:5):

5′-CTATAAACATGCTTTCCGTGGCAGTGAGAACAAATGGGACCGTGCATTGC

(GAGCGTCAGTCTTCGTCCAG)

Target nucleic acid 70-20 (SEQ ID NO:6):

5′-CTATAAACATGCTTTCCGTGGCAGTGAGAA

(GAGCGTCAGTCTTCGTCCAG) CAAATGGGACCGTGCATTGC

Target nucleic acid 70-40 (SEQ ID NO:7):

5'-CTATAAACAT (GAGCGTCAGTCTTCGTCCAG)

GCTTTCCGTGGCAGTGAGAACAAATGGGACCGTGACATTGC

Each sequence in round brackets "()" is a probe-binding site, while the 5'-terminus is labeled with a fluorescence coloring matter. The "Y" moieties in SEQ ID NOS:5, 6 and 7 as target sequences have 0, 20 and 40 bases respectively.

(4) Immobilization of the nucleic acid probes

In this example, a gold substrate was used as the substrate. The gold substrate was dipped in a buffer solution containing the nucleic acid probe C-0, C-10, C-20 or C-30, and left at room temperature for 1 hour. Thereafter, the substrate was washed with distilled water and dried whereby a nucleic acid probe-immobilized gold substrate was prepared.

(5) Hybridization with the target nucleic acids

A buffer solution containing each of the 3 target nucleic acids was subjected to thermal denaturation at 95°C for 5 minutes. Thereafter, the reaction solution was quenched to give a target nucleic acid solution. The nucleic acid probe-immobilized gold substrate having each nucleic acid probe immobilized thereon was dipped in this target nucleic acid solution. The substrate was left at 35°C for 1 hour. Thereafter, each nucleic acid probe-immobilized substrate was washed by dipping the substrate in a nucleic acid-free buffer solution and leaving it at 35°C for 1 hour.

(6) Detection of the hybridized target nucleic acids

By detecting fluorescence intensity derived from the fluorescence coloring matter with which the 5'-terminus of the target nucleic acid had been modified, the presence of the target nucleic acid hybridized with the immobilized nucleic acid probe was detected.

(7) Results

(i) When the target nucleic acid 70-0 was used

FIG. 6 shows the results where the target nucleic acid 70-0 was used. The states of the target nucleic acid 70-0 bound to the nucleic acid probes C-0, C-10, C-20 and C-30 are illustrated in FIG. 6A. In any of the nucleic acid probes, $X \geq Y$. From the detected fluorescence intensity, it was found that the amount of the target nucleic acid 70-0 hybridized with each of the nucleic acid probes C-0, C-10, C-20 and C-30 is almost the same.

(ii) When the target nucleic acid 70-20 was used

FIG. 7 shows the results where the target nucleic acid 70-20 was used. The states of the target nucleic acid 70-20 bound to the nucleic acid probes C-0, C-10, C-20 and C-30 are illustrated in FIG. 7A. In the nucleic acid probes C-0 and C-10, $X < Y$; in the probe C-20, $X = Y$; and in the nucleic acid probe C-30, $X > Y$.

As shown in FIG. 7B, the fluorescence intensity detected was increased as the length of the spacer was increased. Similarly, the amount of the target nucleic acid 70-20 hybridized was increased depending on the length of the spacer, and the hybridization was maximized by using the nucleic acid probe containing the same number of cytosine bases (that is, C20) as the number of bases ranging from the nucleic acid probe-binding site to the 3'-terminus of the target nucleic acid, and the degree of hybridization was almost the same as that attained by using the spacer containing a larger number of bases (FIG. 7B).

(iii) When the target nucleic acid 70-40 was used

When the target nucleic acid 70-40 was used as the target nucleic acid, the states of the target nucleic acid 70-20 to the nucleic acid probes C-0, C-10, C-20 and C-30 are illustrated in FIG. 8A. In any of the nucleic acid probes, X < Y. The amount of the target nucleic acid hybridized with each of the nucleic acid probes was similar, and the hybridization efficiency was low (FIG. 8B).

(iv) Conclusion

From the results described above, it was confirmed that hybridization efficiency is improved when upon hybridization, with the nucleic acid probe, of a target nucleic acid partially containing a target sequence, the spacer used in binding the nucleic acid probe onto a solid-phase carrier satisfies the relationship $X \geqq Y$ wherein X is the length of the spacer and Y is the length of the target nucleic acid ranging from the end of the hybridized site at the side of the substrate to the end of the target nucleic acid at the side of the substrate. By improvement of hybridization efficiency, the presence of the target nucleic acid can be detected more accurately.

(8) Regulation 1 by amplification of a nucleic acid sample by use of primers

According to a further embodiment of this invention, there is provided a method further comprising a step of amplifying a nucleic acid sample by using nucleic acid probes giving a preferable target nucleic acid before the reaction of a nucleic acid sample with the nucleic acid probe-immobilized substrate in accordance with the embodiments of this invention described above. Such nucleic acid probes, which are primers for amplifying the nucleic acid sample, are hybridized with the target sequence in the target nucleic acid such that the end of the target nucleic acid is located within about 40 bases, preferably about 26 to about 12 bases, from the end of the target sequence at the side of the substrate,.

FIG. 9 is a drawing showing the relationship between amplification fragments and a nucleic acid probe according to a further embodiment of this invention. A system for detecting the MxA gene in the human genome is shown in this drawing. FIG. 9 shows the nucleic acid probe in SEQ ID NO:8 and two types of PCR product. The nucleic acid probe in SEQ ID NO:8 has a 20-base spacer (expressed as "spacer-20" in the drawing). PCR product A (referred to hereinafter as "A") was prepared by using a primer biotinated at the 5'-terminus thereof, having the nucleotide sequence in SEQ ID NO:9, and a primer labeled with cys5, having the nucleotide sequence in SEQ ID NO:10. PCR product B (referred to hereinafter as "B") was prepared by using a primer biotinated at the 5'-terminus thereof, having the nucleotide sequence in SEQ ID NO:11, and a primer labeled with cys5, having the nucleotide sequence in SEQ ID NO:12. Preparation of a single strand was carried out by using fine magnetic particles labeled with avidin. In FIG. 9, the distance from the end of the nucleic acid probe-binding site to the end of A is 12 bases (expressed as "12 mer" in the drawing) or the distance to the end of B is 26 bases (expressed as "26 mer" in the drawing). These targets were used to carry out hybridization reaction, and fluorescence intensity was measured. As a result, A showed fluorescence intensity which was 10 times as high as that of B (FIG. 10).

When B was used, the reaction was almost saturated for about 1 hour, while when A was used, the reaction was saturated for about 10 minutes. Further, as a result of examination of specificity for SNP detection, there was an about twice difference in signal-to-noise ratio (S/N) between A and B.

(9) Regulation 2 by amplification of a nucleic acid sample using primers

FIG. 11 is a drawing showing the relationship between amplification fragments and a probe according to a further embodiment of this invention. FIG. 11 shows the results obtained using a nucleic acid probe and amplification products amplified for determination of MBL gene polymorphism in human genome. FIG. 11 shows a nucleic acid probe represented by the nucleotide sequence in SEQ ID NO:13 and three types of PCR products. PCR product C (referred to hereinafter as "C") was prepared by using a primer phosphorylated at the 5'-terminus thereof, having the nucleotide sequence in SEQ ID NO:14 and a primer labeled with cy5, having the nucleotide sequence in SEQ ID NO:15. PCR product D (referred to hereinafter as "D") was prepared by using a primer phosphorylated at the 5'-terminus thereof, having the nucleotide sequence in SEQ ID NO:16 and a primer labeled with cy5, having the nucleotide sequence in SEQ ID NO:15. PCR product E (referred to hereinafter as "E") was prepared by using a primer phosphorylated at the 5'-terminus thereof, having the nucleotide sequence in SEQ ID NO:18 and a primer labeled with cy5, having the nucleotide sequence in SEQ ID NO:17. Further, primers bringing about a PCR product wherein the distance from the end of the nucleic acid probe-binding site to the end of the PCR product was 40 bases were also used. Preparation of a single strand was carried out using $\lambda$-nuclease. As shown in the PCR products in FIG. 11, C has 13 bases (expressed as "13 mer" in the drawing) as the distance from end of the probe-binding site to the end of C, D has 33 bases (expressed as "33 mer" in the drawing) as said distance, and E has 48 bases (expressed as "48 mer" in the drawing) as said distance. Although not shown in the drawing, a PCR product wherein the distance from the end of the probe-binding site to the end of the PCR product was 40 bases was also obtained. By using these targets, hybridization reaction was carried out to measure fluorescence intensity. As a result, C indicated fluorescence intensity which was about 6 times as high as that of D and about 40 times as high as that of E (FIG. 12).

FIG. 13 showed the detection results in an MBL detection system by electrochemical means. After the hybridization reaction with each target, the electric current of Hoechst 33258 was measured. As a result, target C indicated an electric current which was about 3 times as high as that of target D and about at least 10 times as high as that of E.

From the results described above, it was found that when the 3'-terminus of a primer is located at a site apart by 40 bases or more from the center of the nucleic acid probe-binding site, the hybridization efficiency is significantly lowered. Further, a reduction in specificity is also observed, and thus amplification by primers located within 40 bases from the center of the nucleic acid probe-binding site is important for rapid, highly selective and highly sensitive detection of nucleic acid.

According to the invention described above, there are provided a method of detecting a nucleic acid sequence highly sensitively and specifically and primers used therein.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A nucleic acid probe-immobilized substrate comprising a substrate and a nucleic acid probe containing a nucleotide sequence complementary to a target sequence and immobilized via a spacer onto said substrate, wherein upon hybridization, with the nucleic acid probe, of a target nucleic acid partially containing the target sequence, the spacer satisfies the relationship:

$$X \geqq Y$$

wherein X is the length of the spacer and Y is the length of the target nucleic acid ranging from the end of the hybridized site at the side of the substrate to the end of the target nucleic acid at the side of the substrate.

2. A nucleic acid probe-immobilized substrate according to claim 1, wherein the relationship between X and Y is $X \geqq Y$ and $Y \geqq 10$ Å.

3. A nucleic acid probe-immobilized substrate according to claim 1, wherein the relationship between X and Y is $X \geqq Y$, $Y \geqq 10$ Å, and $X-10$ Å $\geqq Y$.

4. A nucleic acid probe-immobilized substrate according to claim 1, wherein the relationship between X and Y is $X \geqq Y$, $Y \geqq 10$ Å, $X-10$ Å $\geqq Y$, and $200$ Å $\geqq X$.

5. A nucleic acid probe-immobilized substrate according to claim 1, wherein the relationship between X and Y is $X \geqq Y$, $Y \geqq 10$ Å, $X-10$ Å $\geqq Y$, $200$ Å $\geqq X$, and $X \geqq 100$ Å.

6. A nucleic acid probe-immobilized substrate according to claim 1, wherein the relationship between X and Y is $X \geqq Y$, $Y \geqq 10$ Å, $X-10$ Å $\geqq Y$, and $100$ Å $\geqq X$.

7. A nucleic acid probe-immobilized substrate according to claim 1, wherein the relationship between X and Y is $X \geqq Y$ and $10$ Å $\geqq Y$.

8. A nucleic acid probe-immobilized substrate according to claim 1, wherein the spacer is an organic linear molecule.

**9.** A nucleic acid probe-immobilized substrate according to claim 1, wherein the spacer is a member selected from the group consisting of nucleic acid, ethylene glycol and alkane.

**10.** A nucleic acid probe-immobilized substrate according to claim 1, wherein the substrate comprises an electrode capable of electrochemical detection, and the nucleic acid probe is immobilized via a spacer onto the electrode.

**11.** A nucleic acid probe-immobilized substrate according to claim 8, wherein the substrate comprises an electrode capable of electrochemical detection, and the nucleic acid probe is immobilized via a spacer onto the electrode.

**12.** A nucleic acid probe-immobilized substrate according to claim 9, wherein the substrate comprises an electrode capable of electrochemical detection, and the nucleic acid probe is immobilized via a spacer onto the electrode.

**13.** A method of detecting the presence of a target nucleic acid by use of the nucleic acid probe-immobilized substrate according to claim 1, the method comprising:

a step of amplifying a nucleic acid sample with primers selected so as to satisfy the relationship $X \geqq Y$;
a step of allowing the amplification product obtained by the amplification to react, under conditions achieving suitable hybridization, with a nucleic acid probe immobilized on the nucleic acid probe-immobilized substrate according to claim 1; and
a step of detecting the hybridization occurring in the above reaction, thereby determining that the target nucleic acid is present in the nucleic acid sample.

**14.** A method of detecting the presence of a target nucleic acid by use of the nucleic acid probe-immobilized substrate according to claim 8, the method comprising:

a step of amplifying a nucleic acid sample with primers selected so as to satisfy the relationship $X \geqq Y$;
a step of allowing the amplification product obtained by the amplification to react, under conditions achieving suitable hybridization, with a nucleic acid probe immobilized on the nucleic acid probe-immobilized substrate according to claim 8; and
a step of detecting the hybridization occurring in the above reaction, thereby determining that the target nucleic acid is present in the nucleic acid sample.

**15.** A method of detecting the presence of a target nucleic acid by use of the nucleic acid probe-immobilized substrate according to claim 9, the method comprising:

a step of amplifying a nucleic acid sample with primers selected so as to satisfy the relationship $X \geqq Y$;
a step of allowing the amplification product obtained by the amplification to react, under conditions achieving suitable hybridization, with a nucleic acid probe immobilized on the nucleic acid probe-immobilized substrate according to claim 9; and
a step of detecting the hybridization occurring in the above reaction, thereby determining that the target nucleic acid is present in the nucleic acid sample.

**16.** A method of detecting the presence of a target nucleic acid by use of the nucleic acid probe-immobilized substrate according to claim 10, the method comprising:

a step of amplifying a nucleic acid sample with primers selected so as to satisfy the relationship $X \geqq Y$;
a step of allowing the amplification product obtained by the amplification to react, under conditions achieving suitable hybridization, with a nucleic acid probe immobilized on the nucleic acid probe-immobilized substrate according to claim 10; and
a step of detecting the hybridization occurring in the above reaction, thereby determining that the target nucleic acid is present in the nucleic acid sample.

**17.** A method of detecting a target nucleic acid containing a target sequence by use of a nucleic acid probe-immobilized substrate comprising a substrate and a nucleic acid probe containing a sequence complementary to the target sequence and immobilized onto said substrate, the method comprising:

(a) preparing primers for amplifying the target nucleic acid such that, upon hybridization of the target sequence in the target nucleic acid with the nucleic acid probe, the end of the target nucleic acid is located within 40 bases from the end of the target sequence at the side of the substrate,
(b) amplifying a nucleic acid sample with the primers prepared in the step (a) above;
(c) allowing the amplification product obtained in the step (b) above to be single-stranded;
(d) allowing the single strand obtained in the step (c) above to react with the nucleic acid probe; and
(e) detecting the hybridization occurring in the step (d) above, thereby detecting the presence of the target nucleic acid in the nucleic acid sample.

**18.** A method of detecting the presence of a target nucleic acid containing a target sequence by use of the nucleic acid probe-immobilized substrate described in claim 1, the method comprising:

(a) preparing primers for amplifying the target nucleic acid such that, upon hybridization of the target sequence in the target nucleic acid with the nucleic acid probe, the end of the target nucleic acid is located within 40 bases from the end of the target sequence at the side of the substrate,
(b) amplifying a nucleic acid sample with the primers prepared in the step (a) above;
(c) allowing the amplification product obtained in the step (b) above to be single-stranded;
(d) allowing the single strand obtained in the step (c) above to react with the nucleic acid probe; and
(e) detecting the hybridization occurring in the step (d) above, thereby detecting the presence of the target nucleic acid in the nucleic acid sample.

**19.** A method of detecting the presence of a target nucleic acid containing a target sequence by use of the nucleic acid probe-immobilized substrate according to claim 8, the method comprising:

(a) preparing primers for amplifying the target nucleic acid such that, upon hybridization of the target sequence in the target nucleic acid with the nucleic acid probe, the end of the target nucleic acid is located within 40 bases from the end of the target sequence at the side of the substrate,
(b) amplifying a nucleic acid sample with the primers prepared in the step (a) above;
(c) allowing the amplification product obtained in the step (b) above to be single-stranded;
(d) allowing the single strand obtained in the step (c) above to react with the nucleic acid probe; and
(e) detecting the hybridization occurring in the step (d) above, thereby detecting the presence of the target nucleic acid in the nucleic acid sample.

**20.** A method of detecting the presence of a target nucleic acid containing a target sequence by use of the nucleic acid probe-immobilized substrate according to claim 10, the method comprising:

(a) preparing primers for amplifying the target nucleic acid such that, upon hybridization of the target sequence in the target nucleic acid with the nucleic acid probe, the end of the target nucleic acid is located within 40 bases from the end of the target sequence at the side of the substrate,
(b) amplifying a nucleic acid sample with the primers prepared in the step (a) above;
(c) allowing the amplification product obtained in the step (b) above to be single-stranded;
(d) allowing the single strand obtained in the step (c) above to react with the nucleic acid probe; and
(e) detecting the hybridization occurring in the step (d) above, thereby detecting the presence of the target nucleic acid in the nucleic acid sample.

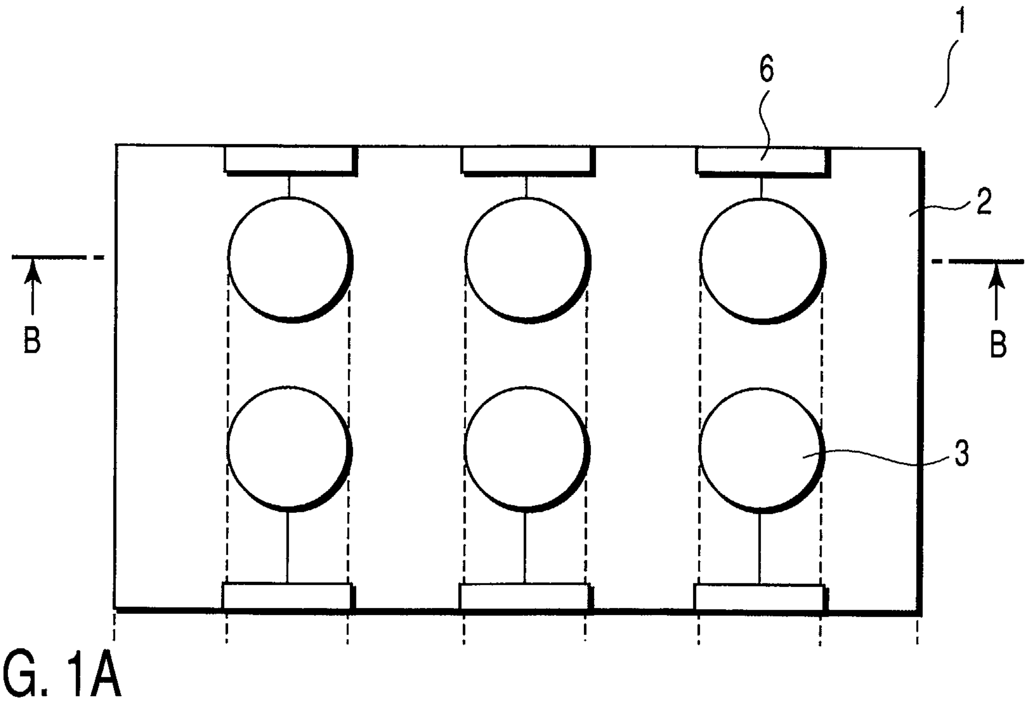


FIG. 1A

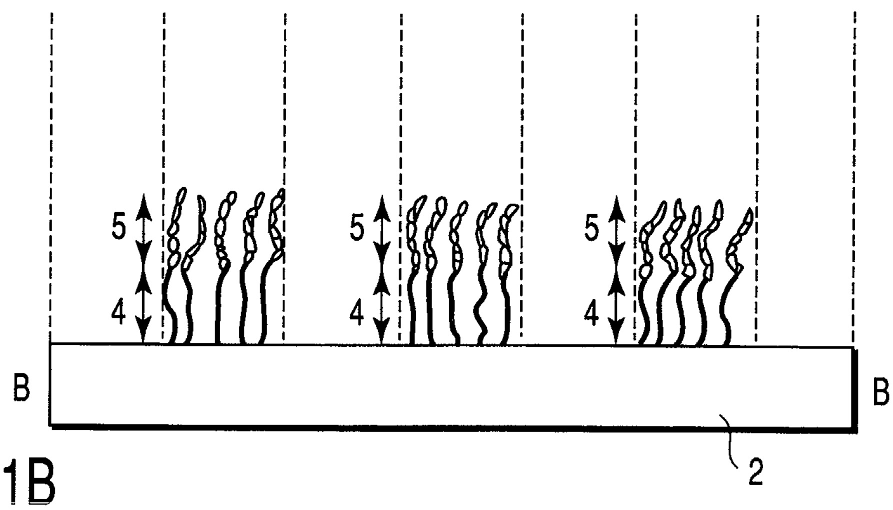


FIG. 1B

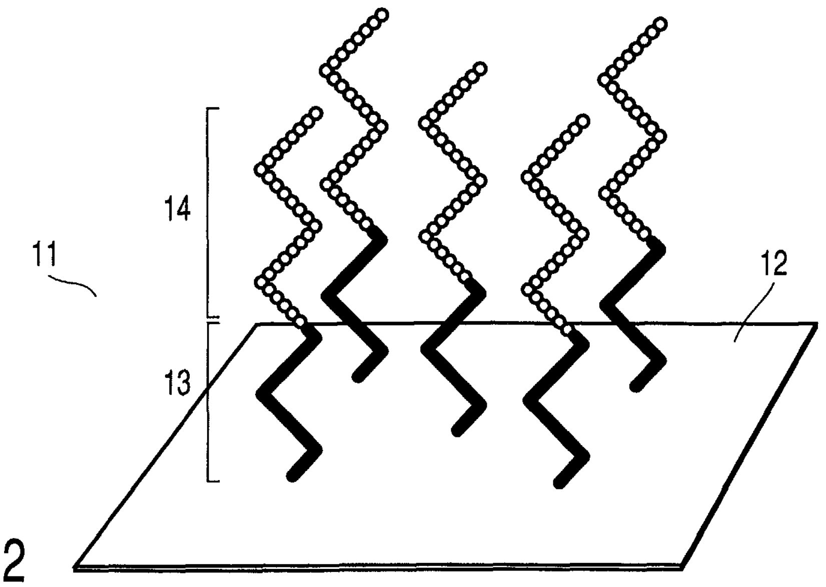
14
11
12
13

FIG. 2

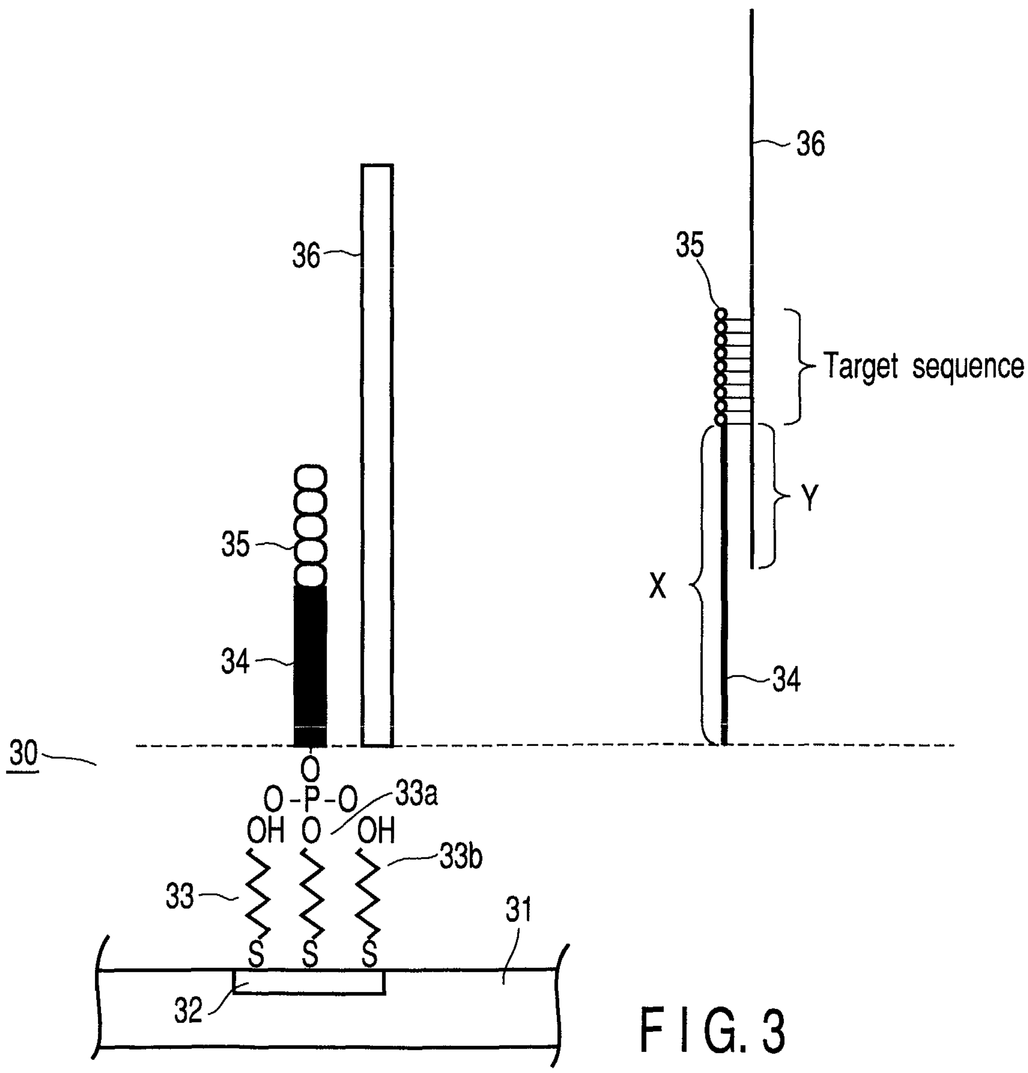
36
36
35
Target sequence
Y
35
X
34
34
30
O
O-P-O
33a
OH O OH
33b
33
31
S S S
32

FIG. 3

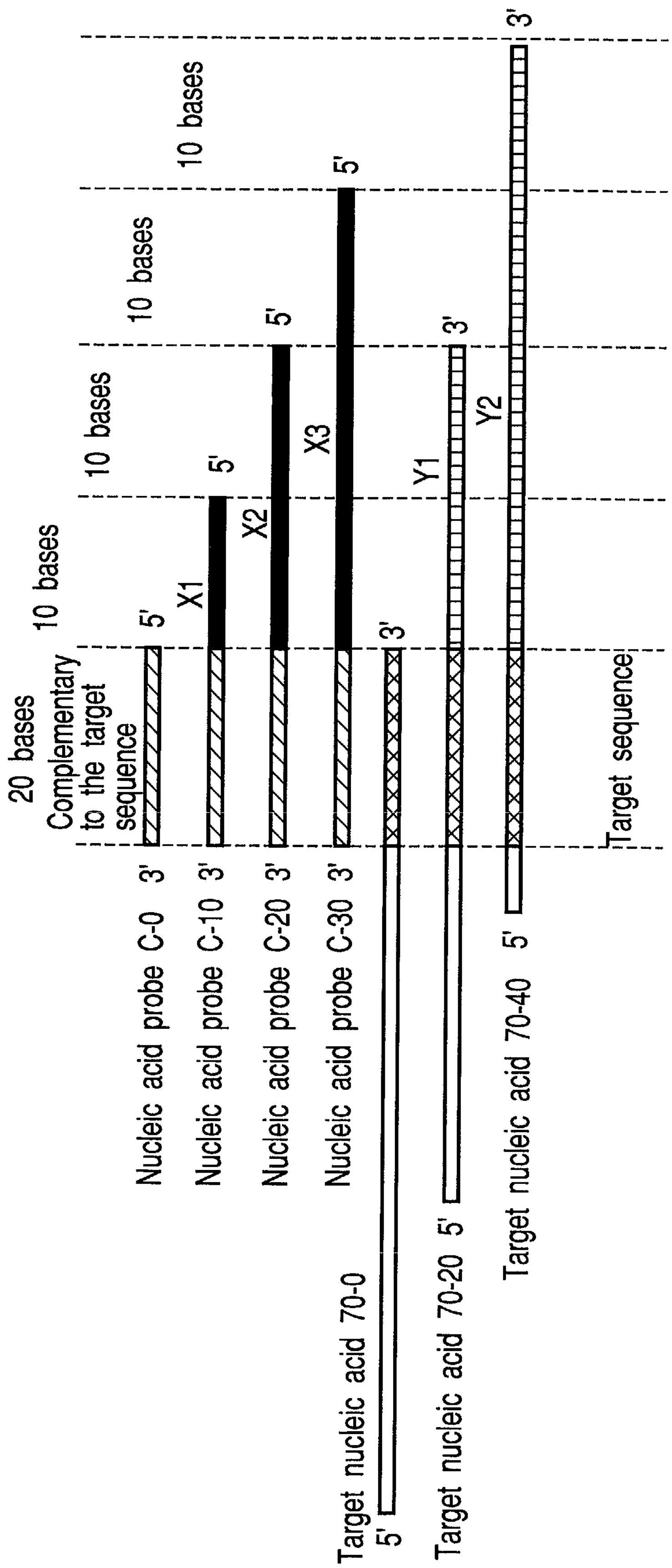
20 bases
Complementary to the target sequence
10 bases
10 bases
10 bases
10 bases
Nucleic acid probe C-0 3' 5'
Nucleic acid probe C-10 3' 5'
Nucleic acid probe C-20 3' 5'
Nucleic acid probe C-30 3' 5'
X1
X2
X3
Target nucleic acid 70-0 5' 3'
Target nucleic acid 70-20 5' 3'
Target nucleic acid 70-40 5' 3'
Y1
Y2
Target sequence

FIG. 4

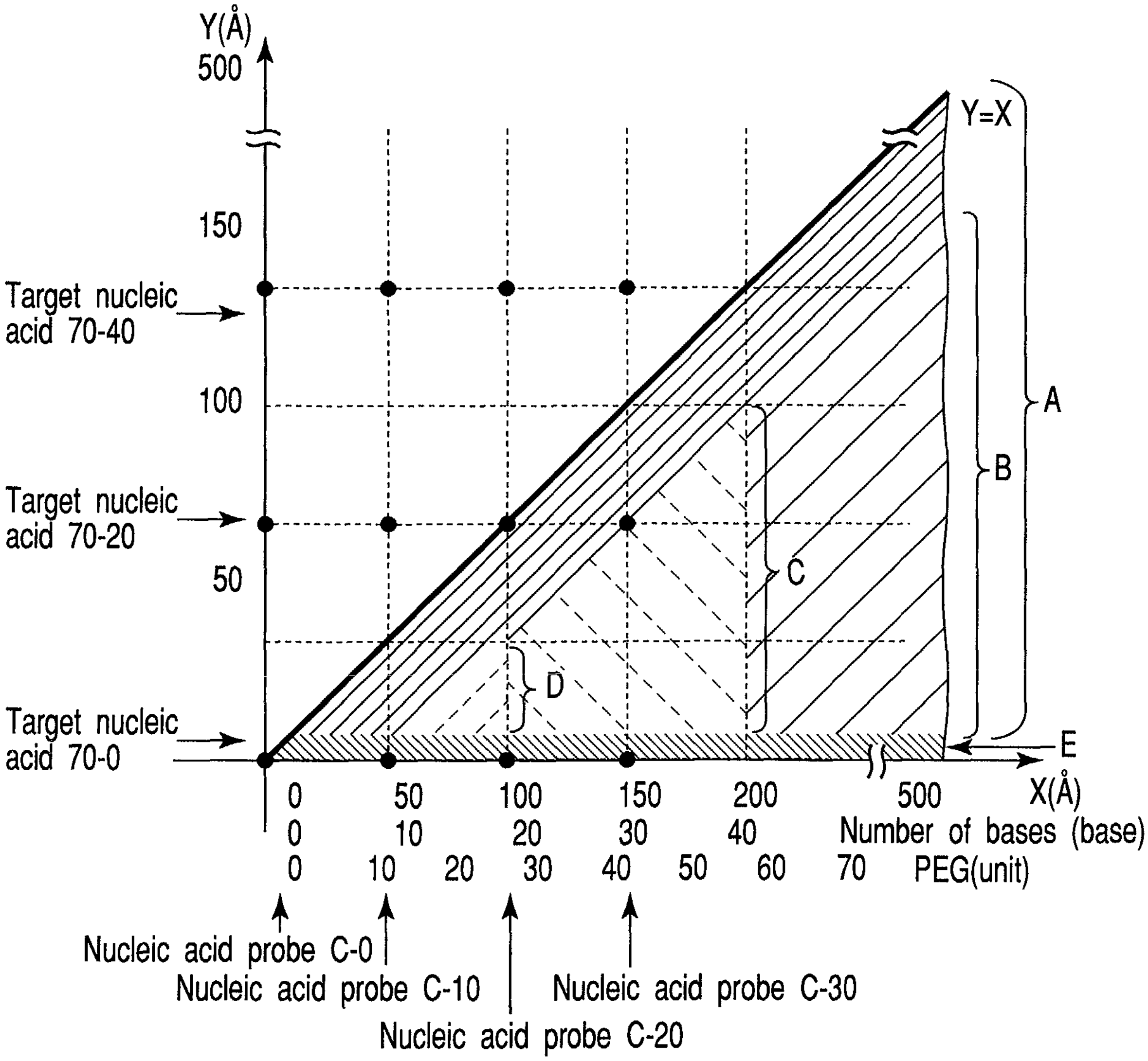

Y(Å)
500
150
Target nucleic acid 70-40
100
Target nucleic acid 70-20
50
Target nucleic acid 70-0
Y=X
A
B
C
D
E
X(Å)
0 50 100 150 200 500
0 10 20 30 40 Number of bases (base)
0 10 20 30 40 50 60 70 PEG(unit)
Nucleic acid probe C-0
Nucleic acid probe C-10
Nucleic acid probe C-20
Nucleic acid probe C-30


FIG. 5

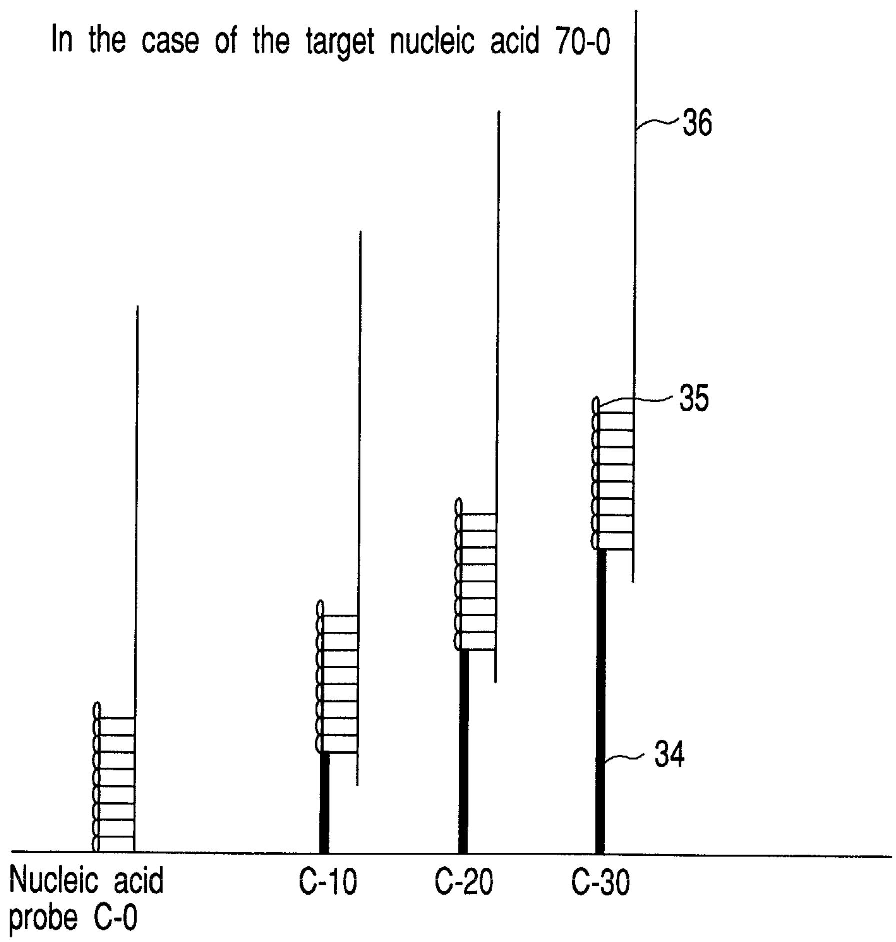
In the case of the target nucleic acid 70-0
36
35
34
Nucleic acid probe C-0
C-10
C-20
C-30

F I G. 6A

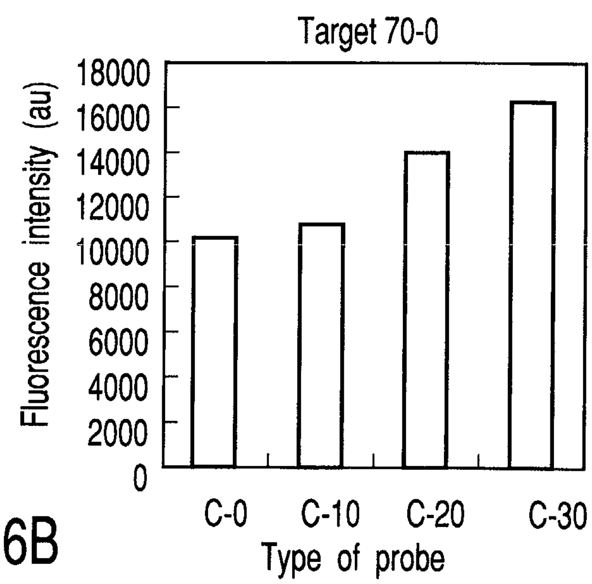
Target 70-0
Fluorescence intensity (au)
18000
16000
14000
12000
10000
8000
6000
4000
2000
0
C-0
C-10
C-20
C-30
Type of probe

F I G. 6B

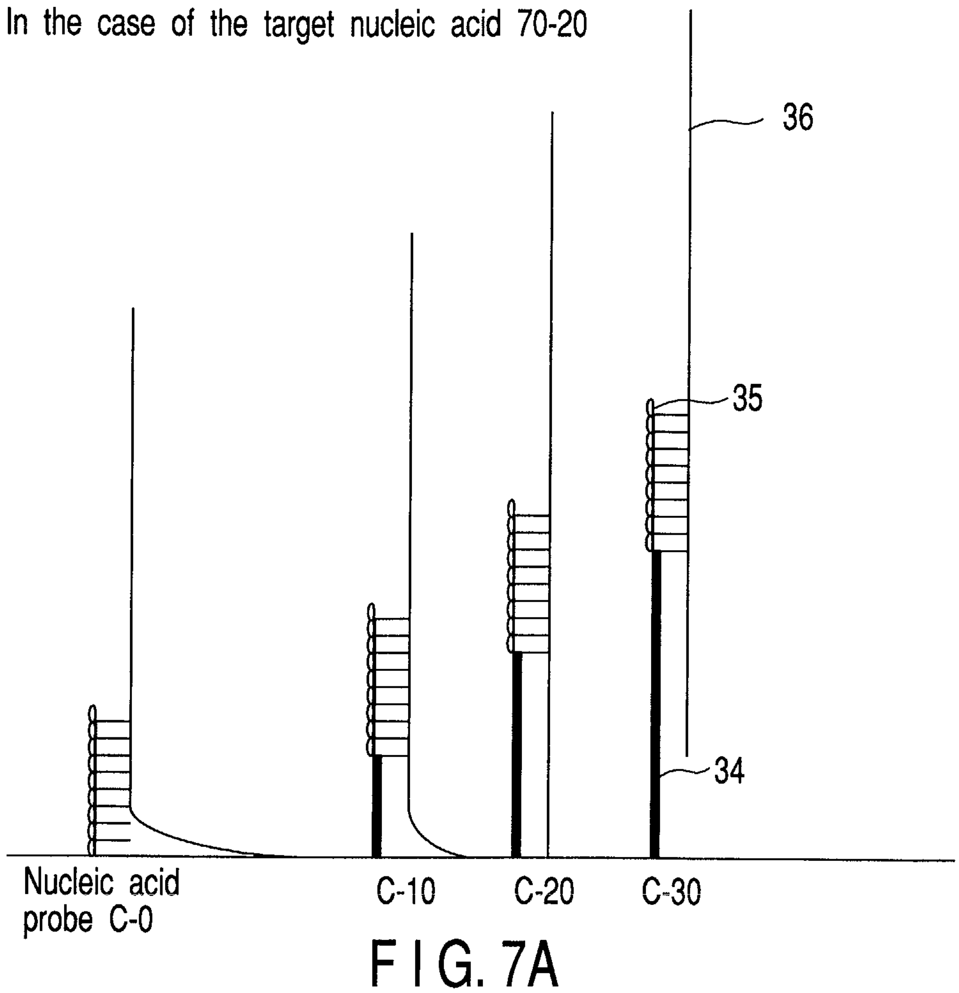
In the case of the target nucleic acid 70-20
36
35
34
Nucleic acid probe C-0
C-10
C-20
C-30

F I G. 7A

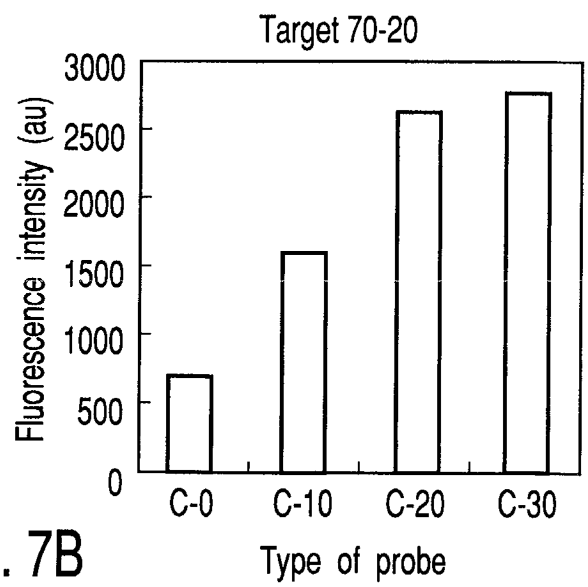
Target 70-20
Fluorescence intensity (au)
3000
2500
2000
1500
1000
500
0
C-0
C-10
C-20
C-30
Type of probe

F I G. 7B

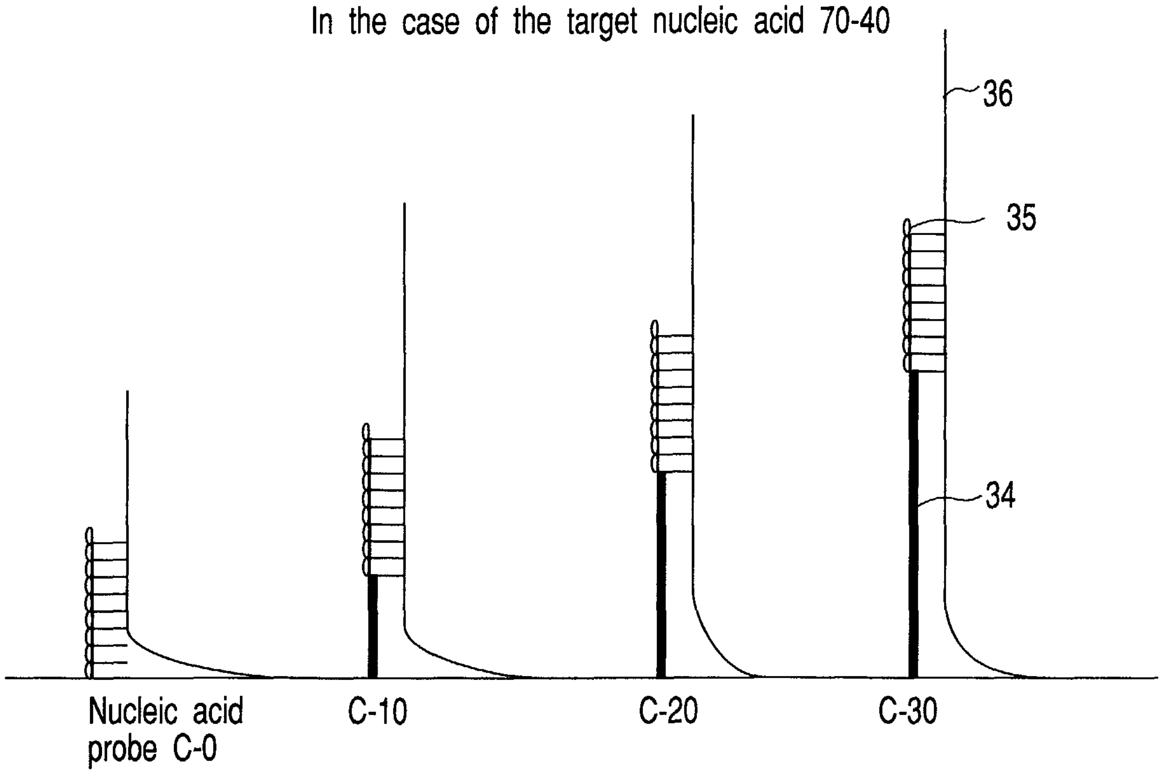
In the case of the target nucleic acid 70-40
36
35
34
Nucleic acid
probe C-0
C-10
C-20
C-30

FIG. 8A

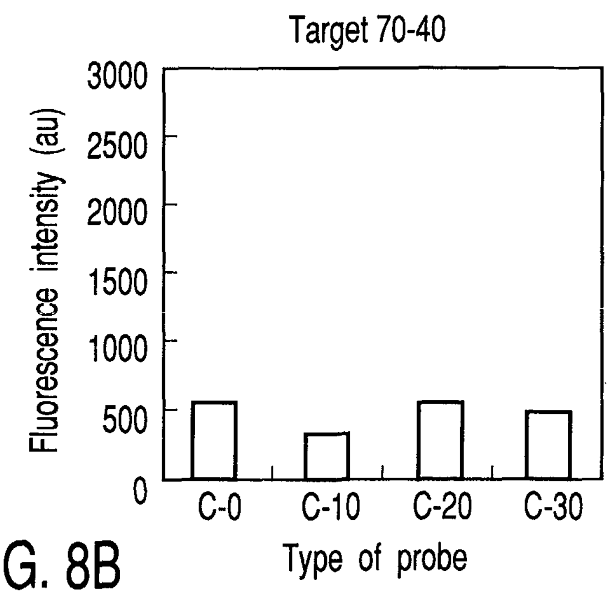
Target 70-40
3000
2500
2000
1500
1000
500
0
Fluorescence intensity (au)
C-0
C-10
C-20
C-30
Type of probe

FIG. 8B

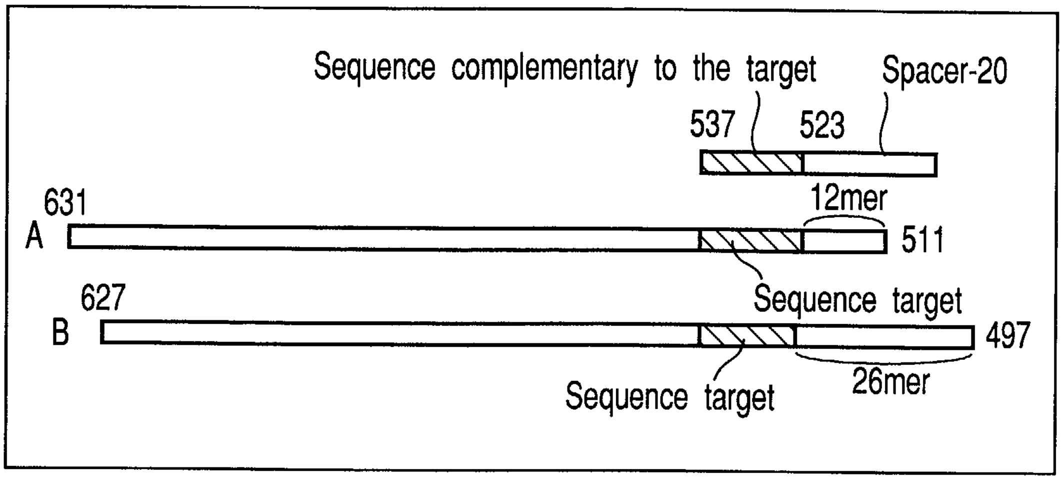
Sequence complementary to the target
Spacer-20
537
523
631
A
12mer
511
627
B
Sequence target
497
Sequence target
26mer

FIG. 9

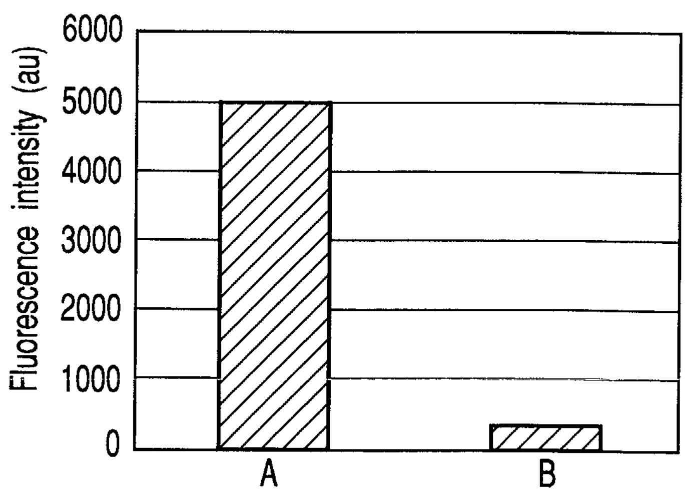
Fluorescence intensity (au)
6000
5000
4000
3000
2000
1000
0
A
B

FIG. 10

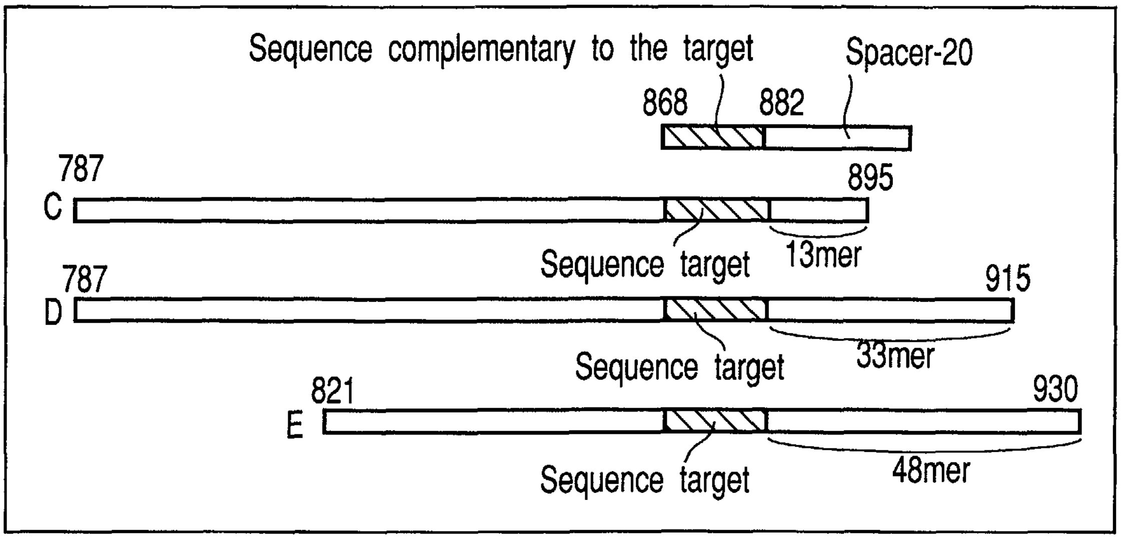
Sequence complementary to the target
Spacer-20
868
882
787
C
895
Sequence target
13mer
787
D
915
Sequence target
33mer
821
E
930
Sequence target
48mer

FIG. 11

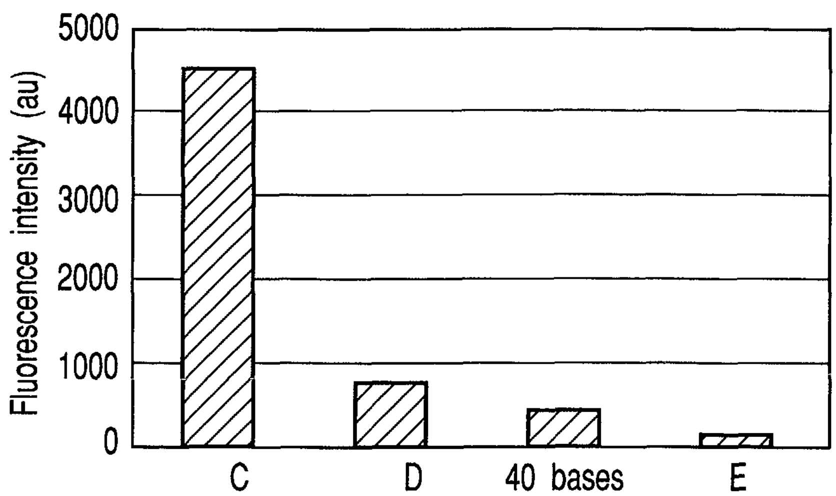
Fluorescence intensity (au)
5000
4000
3000
2000
1000
0
C
D
40 bases
E

FIG. 12

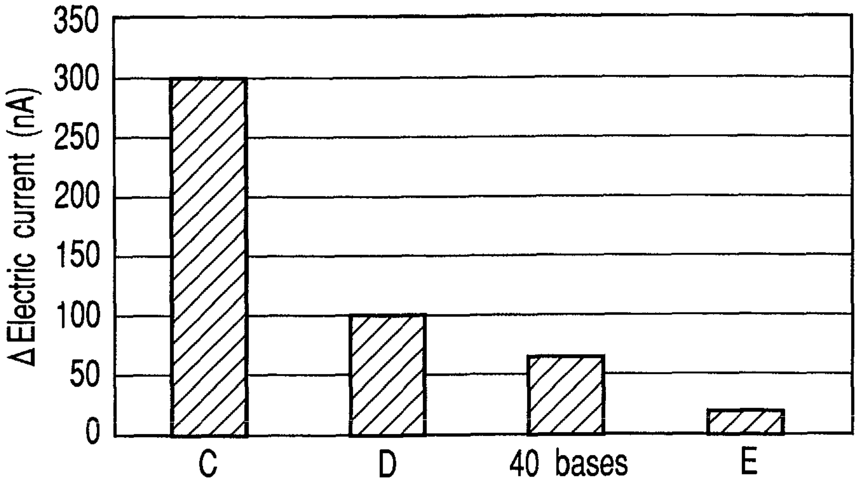


FIG. 13

## INTERNATIONAL SEARCH REPORT

International application No.
PCT/JP02/08670

A. CLASSIFICATION OF SUBJECT MATTER
Int.Cl⁷ C12N15/09, C12Q1/68, G01N33/50

According to International Patent Classification (IPC) or to both national classification and IPC

B. FIELDS SEARCHED

Minimum documentation searched (classification system followed by classification symbols)
Int.Cl⁷ C12N15/09, C12Q1/68, G01N33/50

Documentation searched other than minimum documentation to the extent that such documents are included in the fields searched

Electronic data base consulted during the international search (name of data base and, where practicable, search terms used)
WPI(DIALOG), JICST FILE(JOIS)

C. DOCUMENTS CONSIDERED TO BE RELEVANT

| Category* | Citation of document, with indication, where appropriate, of the relevant passages | Relevant to claim No. |
|---|---|---|
| X | Sanguedolce, L.A. et al., Fundamental Studies of DNA Adsorption and Hybridization on Solid Surfaces. Am.Chem.Soc. Symp.Ser., No.728, 1999, pages 190 to 204 | 1-20 |
| X | WO 89/11548 A1 (Cetus Corp.), 30 November, 1989 (30.11.89), & EP 451141 A1 & JP 3-504328 A | 1-20 |
| X | EP 511559 A1 (F. Hoffmann-La Roche), 04 November, 1992 (04.11.92), & JP 5-168499 A | 1-20 |
| A | WO 96/17955 A2 (Chiron Corp.), 13 June, 1996 (13.06.96), & EP 795033 A1 & US 5736327 A & JP 10-509595 A | 1-20 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 October, 2002 (24.10.02) | 19 November, 2002 (19.11.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/08670

C (Continuation). DOCUMENTS CONSIDERED TO BE RELEVANT

| Category* | Citation of document, with indication, where appropriate, of the relevant passages | Relevant to claim No. |
|---|---|---|
| A | JP 6-98797 A (Toyobo Co., Ltd.), 12 April, 1994 (12.04.94), (Family: none) | 1-20 |
| A | JP 2001-269197 A (Toyobo Co., Ltd.), 02 October, 2001 (02.10.01), (Family: none) | 1-20 |

Form PCT/ISA/210 (continuation of second sheet) (July 1998)

SEQUENCE LISTING

<110> KABUSHIKI KAISHA TOSHIBA
TAKAHASHI, Masayoshi
OKADA, Jun
HASHIMOTO, Koji

<120> The nucleic acids probe immobilized substrate and Method for detecting of nucleic acid with it

<130> 02S1022P

<150> JP 2002-218644

<151> 2002-7-26

<160> 18

<210> 1
<211> 19
<212> DNA
<213> E. Coli
<400> 1
TGGACGAAGA CTGACGCTC 19

<210> 2
<211> 29
<212> DNA

<213> artificial

<400> 2

CCCCCCCCCC TGGACGAAGA CTGACGCTC 29

<210> 3

<211> 39

<212> DNA

<213> artificial

<400> 3

CCCCCCCCCC CCCCCCCCCC TGGACGAAGA CTGACGCTC 39

<210> 4

<211> 45

<212> DNA

<213> artificial

<400> 4

CCCCCCCCCC CCCCCCCCCC CCCCCCCCCC TGGACGAAGA CTGACGCTC 45

<210> 5

<211> 70

<212> DNA

<213> artificial

<400> 5

CTATAAACAT GCTTTCCGTG GCAGTGAGAA CAAATGGGAC CGTGCATTGC GAGCGTCAGT 60

CTTCGTCCAG 70

<210> 6

<211> 70

<212> DNA

<213> artificial

<400> 6

CTATAAACAT GCTTTCCGTG GCAGTGAGAA GAGCGTCAGT CTTCGTCCAG CAAATGGGAC 60

CGTGCATTGC 70

<210> 7

<211> 70

<212> DNA

<213> artificial

<400> 7

CTATAAACAT GAGCGTCAGT CTTCGTCCAG GCTTTCCGTG GCAGTGAGAA CAAATGGGAC 60

CGTGCATTGC 70

<210> 8

<211> 35

<212> DNA

<213> artificial sequence

<400> 8

CCCCCCCCCC CCCCCCCCCC GTTTCTGCTC CCGGA 35

<210> 9

<211> 20

<212> DNA

<213> homo sapience

<400> 9

GAGCTAGGTT TCGTTTCTGC 20

<210> 10

<211> 22

<212> DNA

<213> homo sapience

<400> 10

GGCCTCCGCT CTCGCTTCGC CT 22

<210> 11

<211> 22

<212> DNA

<213> homo sapience

<400> 11

AGGTGCGGGG CCAGGAGCTA GG 22

<210> 12

<211> 20

<212> DNA

<213> homo sapience

<400> 12

TCCGCTCTCG CTTCGCCTCT 20

<210> 13

<211> 35

<212> DNA

<213> artificial sequence

<400> 13

CCCCCCCCCC CCCCCCCCCC CTTGGTGTCA TCACG 35

<210> 14

<211> 20

<212> DNA

<213> homo sapience

<400> 14

CCCCCTTTTC TCCCTTGGTG 20

<210> 15

<211> 20

<212> DNA

<213> homo sapience

<400> 15

TGTGAGGATG CCCAAAAGAC 20

<210> 16

<211> 20

<212> DNA

<213> homo sapience

<400> 16

AGCCCAACAC GTACCTGGTT 9

<210> 17

<211> 20

<212> DNA

<213> homo sapience

<400> 17

TTGCCTGTAG CTCTCCAGGC 20

<210> 18

<211> 20

<212> DNA

<213> homo sapience

<400> 18

TTGCAGAGAC AGAACAGCCC 20